# EUROPEAN PATENT APPLICATION

(11) **EP 4 431 154 A2**
(43) Date of publication of application: **18.09.2024**
(21) Application number: 24173629.7
(22) Date of filing: 11.02.2019
(51) Int. Cl.: A61P 35/00

(54) **OSIMERTINIB FOR USE IN THE TREATMENT OF NON-SMALL CELL LUNG CANCER**

(30) Priority: 12.02.2018 US 201862629166 P
(62) Divisional of application: 19705720.1
(71) Applicant: Astrazeneca AB, 151 85 Södertälje (SE)
(72) Inventor: NASH, Anthony, Francis, Patrick, Cambridge, SG8 6HB (GB)
(74) Representative: AstraZeneca Intellectual Property

(57) **Abstract**

The specification relates to osimertinib or a pharmaceutically acceptable salt thereof for use in the treatment of patients with locally advanced unresectable epidermal growth factor receptor (EGFR) mutation-positive non-small cell lung cancer (Stage III)), and in particular to the treatment of patients whose disease has not progressed following definitive platinum-based chemoradiation therapy.

## Description

### FIELD OF THE INVENTION

This specification describes osimertinib or a pharmaceutically acceptable salt thereof for use in the treatment of patients with locally advanced unresectable epidermal growth factor receptor (EGFR) mutation-positive non-small cell lung cancer (Stage III). In particular, the specification describes such treatment in patients whose disease has not progressed following definitive platinum-based chemoradiation therapy.

### BACKGROUND OF THE INVENTION

Primary lung cancer is the most common form of cancer (12.9% of all new cancers worldwide) after non melanocytic skin cancer and it remains the leading cause of cancer related death overall (19.4% of all deaths from cancer) (Ann. Oncol. [2014], vol. 25 (Suppl. 3), 27-39). Non small cell lung cancer (NSCLC) represents approximately 80% to 85% of all lung cancers.

Epidermal growth factor receptor (EGFR) tyrosine kinase inhibitors (TKIs) such as gefitinib, erlotinib, lapatinib, and afatinib typically prolong the progression-free survival of patients with metastatic non-small cell lung cancer (NSCLC) whose tumours display activating mutation(s) in the EGFR gene. The most common EGFR activating mutations are L858R substitution mutations and exon 19 deletions. However, the vast majority of such patients develop resistance to these EGFR-TKIs after treatment, usually within 9 to 13 months. One important mechanism of acquired resistance is the T790M EGFR mutation in exon 20 of the EGFR gene. This acquired resistance has led to the development of further EGFR-TKIs such as osimertinib (TAGRISSO^{™}), which is approved for the treatment of patients with metastatic EGFR T790M mutation-positive NSCLC, who have progressed on or after alternative EGFR-TKI therapy.

However, although EGFR-TKIs have significantly improved the prognosis for NSCLC patients in the metastatic setting, these drugs do not represent the standard of care for patients with Stage III unresectable NSCLC. Stage III NSCLC is estimated to affect around 100,000 patients in the G7 countries (US, France, Germany, Italy, Spain, the United Kingdom and Japan) in 2016, the majority of whom (~70%) have unresectable disease (Kantar Health Cancer MPact 2016). Based on a US National Cancer Database of 813,032 patients diagnosed with NSCLC between 1998 and 2006 and with available staging information, the proportion of patients diagnosed with Stage III disease was approximately 27% (J. Thorac. Oncol. [2010], 29-33).

The current standard of care for patients with unresectable Stage III NSCLC is treatment with curative intent with chemoradiation. The same standard of care applies both to EGFR mutation positive and EGFR wild-type patients. Concurrent chemoradiation (CCRT) is considered the standard of care for younger patients with good performance status and minimal comorbidities. Sequential chemoradiation (SCRT) is generally given for less fit patients. However, the difference in the level of efficacy is small.

For EGFR unselected patients, improvements in (i) tumor staging (leading to stage migration); (ii) planning and delivery of radiotherapy, eg, intensity-modulated radiotherapy (IMRT); and (iii) management of toxicities associated with chemoradiation, has led to some improvements in outcomes for patients with unresectable disease. Efficacy outcomes associated with CCRT have ranged from a median overall survival (OS) of 20.6 to 28.7 months (Lancet Oncol. [2015], vol. 16(2), 187-199, J. Clin. Oncol. [2016], vol. 34(9), 953-962, Ann. Oncol. [2017], vol. 28(4), 777-783, J. Clin. Oncol. [2015], vol. 33(24), 2660-2666, Ann. Oncol. [2015], vol. 26(6), 1134-1142).

For patients with unresectable Stage III EGFR mutation-positive NSCLC, the same standard of care of chemoradiation with curative intent is given. In comparison with patients with EGFR wild-type tumours, the median progression-free survival (PFS) following chemoradiation is similar or lower. However within this, locoregional control is superior and distant control inferior (J. Radiat. Res. [2016], vol. 57(5), 449-459). Median overall survival is generally longer in patients with EGFR mutation positive tumours, likely due in part to administration of EGFR-TKIs at the time of disease progression. Following chemoradiation, the standard of care is observation. Patients with EGFR mutation positive NSCLC would only be expected to receive EGFR-TKI following disease progression.

Whilst definitive chemoradiation therapy is given as a treatment of curative intent, unfortunately the majority of patients relapse. There therefore remains a high unmet medical need for patients with unresectable Stage III NSCLC.

Although a number of previous clinical trials have investigated the use of first-generation EGFR-TKIs such as erlotinib, gefitinib and afatinib for the treatment of Stage III NSCLC, the results have been very mixed. These trials are generally small and there is marked heterogeneity with respect to disease characteristics at baseline, prior chemoradiation regimen employed, the timing of the EGFR-TKI treatment, and whether additional treatment strategies are permitted, such as surgery (J Clin Oncol [2008], vol. 26(15), 2450-2456; J Thorac Oncol [2010] vol. 5(9), 1382-1390; Int J Radiat Oncol Biol Phys [2015], vol. 92(2), 317-324; Int JRadiat Oncol Biol Phys [2016] vol. 96(2), E455; J Clin Oncol [2017], vol. 35(15 Suppl), 8531; and Cancer Res Treat [2017], vol. 49(4), 981-989).

There have been no reported Phase III trials investigating the outcome of chemoradiation in patients with EGFR mutation-positive NSCLC. Current International Guidelines indicate that there is no role for EGFR-TKIs in the management of Stage III unresectable EGFR mutation-positive NSCLC. Given the poor prognosis for these patients, there is a clear need for alternative treatment options for patients in this setting. We have found that the use of osimertinib in such patients following definitive platinum-based chemoradiation therapy may result in an improved prognosis, for example an improvement in one or more of improved progression free survival (PFS), or improved duration of response (DoR), or improved overall survival (OS).

### SUMMARY OF THE INVENTION

The present specification describes osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy.

The specification further describes such treatment where osimertinib is administered to the patient only after completion of the definitive platinum-based chemoradiation therapy.

The specification further describes such treatment wherein the treatment results in one or more of improved progression free survival (PFS); improved duration of response (DoR); or improved overall survival (OS).

### DETAILED DESCRIPTION OF THE INVENTION

In a first embodiment, there is provided osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy.

In a further embodiment, there is provided a method of treating locally advanced unresectable EGFR mutation-positive NSCLC (Stage III) in a human patient comprising administering to the patient osimertinib or a pharmaceutically acceptable salt thereof, wherein the disease has not progressed following definitive platinum-based chemoradiation therapy.

In a further embodiment, there is provided the use of osimertinib or a pharmaceutically acceptable salt thereof in the manufacture of a medicament for the treatment of patients with locally advanced unresectable EGFR mutation-positive NSCLC (Stage III), whose disease has not progressed following definitive platinum-based chemoradiation therapy.

As used herein, the term "about" when referring to any given numerical value means within ±10% of that value.

### Locally advanced (Stage III) unresectable NSCLC

Locally advanced (Stage III) NSCLC represents a heterogeneous group of patients with a wide range of prognoses (see for example the European Society of Medical Oncology's guidance on locally advanced stage III non-small-cell lung cancer (Annals of Oncology [2015], vol. 26, 1573-1588, 2015), and the 8th Edition of the International Association for the Study of Lung Cancer (IASLC)/Union for International Cancer Control tumor/node/metastasis (TNM) staging classification (J. Thorac. Oncol. [2016], vol. 11, 39-51)). At one end of the spectrum, patients with T3 and N1 disease are generally considered resectable (i.e., the visible tumour may be removed with surgery). At the other end of the spectrum, patients with T4 disease, ie, with disease that invades vital central structures (diaphragm, mediastinum, heart, great vessels, trachea, recurrent laryngeal nerve, eosophagus, vertebral body, and carina) and/or patients with ipsilateral mediastinal nodal involvement (N2), ipsilateral distant nodal disease, or contralateral nodal involvement (N3), are generally consider unresectable. The various combinations of T stage and nodal status determine whether disease is classified as Stage IIIA, IIIB or IIIC, which are associated with progressively poorer outcomes. The methods of classification of a given patient's locally advanced NSCLC as Stage IIIA, Stage IIIB or Stage IIIC, and the methods of assessing a given patient's localy advanced NSCLC as either resectable or unresectable, would be known to a person skilled in the art.

In one embodiment, therefore, the locally advanced unresectable NSCLC comprises unresectable NSCLC which is considered curable by chemoradiation therapy.

In a further embodiment, the locally advanced unresectable NSCLC comprises locally advanced, non-metastatic NSCLC.

In a further embodiment, the locally advanced unresectable NSCLC comprises Stage IIIC unresectable NSCLC.

In a further embodiment, the locally advanced unresectable NSCLC comprises Stage IIIB unresectable NSCLC.

In a further embodiment, the locally advanced unresectable NSCLC comprises Stage IIIA unresectable NSCLC.

In a further embodiment, the locally advanced unresectable NSCLC comprises Stage IIIB and Stage IIIC unresectable NSCLC.

### EGFR mutation positive NSCLC and diagnostic methods

In 2004 it was reported that activating mutations in exons 18-21 of EGFR correlated with a response to EGFR-TKI therapy in NSCLC (Science [2004], vol. 304, 1497-1500; New England Journal of Medicine [2004], vol. 350, 2129-2139). It is estimated that these mutations are prevalent in approximately 10-16% of NSCLC patients in the United States and Europe, and in approximately 30-50% of NSCLC patients in Asia. Two of the most significant EGFR activating mutations are the exon 19 deletions and the missense mutations in exon 21. The exon 19 deletions account for approximately 45% of known EGFR mutations. Eleven different mutations, resulting in deletion of three to seven amino acids, have been detected in exon 19, and all are centered around the uniformly deleted codons for amino acids 747-749. The most significant exon 19 deletion is E746-A750. The missense mutations in exon 21 account for approximately 39-45% of known EGFR mutations, of which the substitution mutation L858R accounts for approximately 39% of the total mutations in exon 21 (J. Thorac. Oncol. [2010], 1551-1558). The skilled person will be aware of the mutations in EGFR which correlate with an improved response to EGFR-TKI therapy.

In one embodiment, therefore, the EGFR mutation-positive NSCLC comprises activating mutations in EGFR. In a further embodiment, the activating mutations in EGFR comprise activating mutations in exons 18-21. In a further embodiment, the activating mutations in EGFR comprise exon 19 deletions or missense mutations in exon 21. In a further embodiment, the activating mutations in EGFR comprise exon 19 deletions or L858R substitution mutations.

There are numerous methods to detect EGFR activating mutations, of which the skilled person will be aware. These include both tumour tissue and plasma based diagnostic methods. In general, the EGFR mutation status is first assesed using a tumour tissue biopsy sample derived from the patient. If a tumour sample is unavailable, or if the tumour sample is negative, the EGFR mutation status may be assessed using a plasma sample. A particular example of a suitable diagnostic method to detect EGFR activating mutations, and in particular to detect exon 19 deletions or L858R substitution mutations, is the Cobas^{™} EGFR Mutation Test v2 (Roche Molecular System).

In one embodiment, therefore, the EGFR mutation-positive NSCLC comprises activating mutations in EGFR (such as activating mutations in exons 18-21, for example exon 19 deletions or missense mutations in exon 21, for example exon 19 deletions or L858R substitution mutations), wherein the EGFR mutation status of the patient has been determined using an appropriate diagnostic method. In a further embodiment, the EGFR mutation status has been determined using a tumour tissue sample. In a further embodiment, the EGFR mutation status has been determined using a plasma sample. In a further embodiment, the diagnostic method uses the Cobas^{™} EGFR Mutation Test (v1 or v2).

### Definitive platinum-based chemoradiation therapy

The current standard of care for patients with locally advanced unresectable NSCLC (Stage III) is definitive chemoradiation therapy (CRT), i.e. the use of CRT with curative intent rather than for palliative purposes, and in particular definitive platinum-based chemoradiation therapy.

In one embodiment, therefore, the patient comprises a patient with unresectable NSCLC whose disease is considered curable by chemoradiation therapy.

The maximum radiation dose which can be administered in CRT depends upon the ability to target the tumour volume and minimise exposure to healthy tissues, especially exposure to normal healthy organs. Improvements in radiation treatments, such as the introduction of three-dimensional conformal planning, four-dimensional planning CT scans, intensity modulated radiation therapy (IMRT) and image-guided radiation therapy (IGRT) techniques have improved the targeting of the tumour volume thereby allowing higher radiation dosages. It is now possible to safely administer total radiation doses of up to around 100 Gy (Curr. Opin. Oncol. [2011], vol. 23, 140-149). The skilled person will be aware of the appropriate total radiation dose, and appropriate dosing frequency, for any given NSCLC patient.

A number of different chemotherapy agents are suitable for platinum-based CRT use. The standard of care is to give platinum-based doublet chemotherapy, wherein a platinum-based agent (such as cisplatin or carboplatin) is administered in combination with a second, or further, chemotherapy agent. The second, or further, chemotherapy agent may be non-platinum based (such as etoposide, vinorelbine, pemetrexed, a taxane (such as paclitaxel or docetaxel), vinblastine, doxorubicin or gemcitabine). The skilled person will be aware of the appropriate chemotherapy regimen for any given Stage III unresectable NSCLC patient.

There are a number of options for the sequencing of the radiation and chemotherapy treatments in CRT. Treatment may be sequential, wherein CRT is delivered over the course of two separate phases, a first phase in which chemotherapy is administered, followed by a second phase in which radiotherapy is administered (Sequential Chemoradiation Therapy or SCRT). Treatment may also be concurrent, wherein both radiotherapy and chemotherapy are administered concurrently (for example by simultaneous (same day) administration) during a single phase (Concurrent Chemoradiation Therapy or CCRT). Such concurrent treatment may also include an additional phase, either (i) before the concurrent phase, wherein the chemotherapy is further administered in an induction phase, or (ii) after the concurrent phase, wherein chemotherapy is further administered in a consolidation phase, or (iii) both before and after the concurrent phase, wherein the chemotherapy is further administered in both an induction and a consolidation phase. CCRT is considered the standard of care for younger patients with good performance status and minimal comorbidities. SCRT is generally given for less fit patients. Each phase of treatment as described above may comprise one of more individual treatment cycles, as is further described below.

The type of chemotherapy treatment and radiation treatment, and the order of sequencing of the chemotherapy treatment and the radiation treatment, may be an important factor in how a given patient responds to EGFR-TKI treatment.

In one embodiment, therefore, the chemoradiation therapy comprises concurrent chemoradiation therapy (CCRT).

In a further embodiment, the chemoradiation therapy comprises sequential chemoradiation therapy (SCRT).

In a further embodiment, the final chemotherapy cycle ends prior to, or concurrently with, the final dose of radiation.

In a further embodiment, the chemoradiation therapy comprises an induction phase.

In a further embodiment, the chemoradiation therapy comprises a consolidation phase.

In a further embodiment, the concurrent chemoradiation therapy comprises either (i) a treatment period comprising at least 2 treatment cycles wherein chemotherapy is administered concurrently with radiation (such as simultaneous (same day) administration), or (ii) a treatment period comprising at least 5 weekly doses of chemoradiation therpay wherein chemotherapy is administered concurrently with radiation (such as simultaneous (same day) administration).

In a further embodiment, the sequential chemoradiation therapy comprises at least 2 treatment cycles of chemotherapy treatment prior to treatment with radiation.

In a further embodiment, the total dose of radiation administered to the patient as part of the chemoradiation therapy is up to about 100 Gy. In a further embodiment, the total dose of radiation administered to the patient is up to about 90 Gy. In a further embodiment, the total dose of radiation administered to the patient is up to about 80 Gy. In a further embodiment, the total dose of radiation administered to the patient is up to about 70 Gy. In a further embodiment, the total dose of radiation administered to the patient is up to about 60 Gy.

In one embodiment, the platinum-based chemotherapy administered to the patient comprises a single platinum-based agent (such as cisplatin or carboplatin).

In another embodiment, the platinum-based chemotherapy comprises a platinum-based doublet chemotherapy.

In another embodiment, the doublet chemotherapy comprises cisplatin and a second chemotherapy selected from etoposide, vinorelbine, vinblastine, pemetrexed, a taxane (such as paclitaxel or docetaxel), or gemcitabine. In a further embodiment, the doublet chemotherapy comprises cisplatin and a second chemotherapy selected from etoposide, vinorelbine, vinblastine, pemetrexed, or a taxane (such as paclitaxel or docetaxel). In a further embodiment, the doublet chemotherapy comprises cisplatin plus etoposide, or cisplatin plus vinorelbine, or cisplatin plus vinblastine, or cisplatin plus pemetrexed, or cisplatin plus a taxane (such as cisplatin plus paclitaxel, or cisplatin plus docetaxel), or cisplatin plus gemcitabine.

In another embodiment, the doublet chemotherapy comprises carboplatin and a second chemotherapy selected from etoposide, vinorelbine, vinblastine, pemetrexed, a taxane (such as paclitaxel or docetaxel), or gemcitabine. In a further embodiment, the doublet chemotherapy comprises carboplatin and a second chemotherapy selected from etoposide, vinorelbine, vinblastine, pemetrexed, or a taxane (such as paclitaxel or docetaxel). In a further embodiment, the doublet chemotherapy comprises carboplatin plus etoposide, or carboplatin plus vinorelbine, or carboplatin plus vinblastine, or carboplatin plus pemetrexed, or carboplatin plus a taxane (such as carboplatin plus paclitaxel, or carboplatin plus docetaxel), or carboplatin plus gemcitabine.

The total daily dose of cisplatin is generally calculated by reference to Body Surface Area (BSA), and the daily dose typically ranges from between about 50 mg/m² to about 100 mg/m². In one embodiment, therefore, the maximum daily dose of cisplatin is up to about 150 mg/m², such as up to about 120 mg/m², such as up to about 100 mg/m², such as up to about 90 mg/m², such as up to about 80 mg/m², such as up to about 70 mg/m², such as up to about 60 mg/m², such as up to about 50 mg/m².

Patients undergoing CRT with cisplatin do not generally receive cisplatin daily and cisplatin is generally administered in treatment cycles. In one embodiment, the treatment cycle is up to 42 days, such as up to 35 days, such as up to 28 days, such as up to 21 days. In one embodiment, cisplatin is administered on day 1 of each treatment cycle. In one embodiment, cisplatin is administered on day 1 only of a treatment cycle lasting 21 days. In one embodiment, cisplatin is administered on day 1 only of a treatment cycle lasting 28 days. In one embodiment, cisplatin is administered on days 1 and 8 of a treatment cycle lasting 28 days. In one embodiment, cisplatin is administered on days 1 and 29 of a treatment cycle lasting 35 days. In one embodiment, cisplatin is administered on days 1, 8, 29 and 36 of a treatment cycle lasting 42 days.

The total daily dose of carboplatin is generally calculated by reference to the Area Under the Curve (AUC) for a given patient using a formula known to those skilled in the art (such as the Calvert Formula). The typical daily dose ranges from AUC 2 to AUC 6. In one embodiment, the maximum daily dose of carboplatin is up to AUC 6, such as up to AUC 5, such as up to AUC 4, such as up to AUC 3, such as up to AUC 2.

Patients undergoing CRT with carboplatin do not generally receive carboplatin daily and carboplatin is generally administered in treatment cycles. In one embodiment, the treatment cycle is up to 21 days, such as up to 14 days, such as up to 7 days. In one embodiment, cisplatin is administered on day 1 of each treatment cycle. In one embodiment, cisplatin is administered on day 1 only of a treatment cycle lasting 7 days. In one embodiment, cisplatin is administered on day 1 only of a treatment cycle lasting 21 days.

The total daily dose of the non-platinum based chemotherapeutic agent is generally calculated by reference to Body Surface Area (BSA).

In one embodiment, the total daily dose of etoposide is from about 50 mg/m² to about 100 mg/m², such as up to about 100 mg/m², such as up to about 90 mg/m², such as up to about 80 mg/m², such as up to about 70 mg/m², such as up to about 60 mg/m², such as up to about 50 mg/m². In a further embodiment, etoposide is administered on days 1, 2 and 3 of a treatment cycle lasting 21 days. In a further embodiment, etoposide is administered on days 1, 2, 3, 4, 5, 29, 20, 31, 32 and 33 of a treatment cycle lasting 42 days.

In one embodiment, the total daily dose of vinblastine is from about 3 mg/m² to about 20 mg/m², such as up to about 20 mg/m², such as up to about 15 mg/m², such as up to about 10 mg/m², such as up to about 5 mg/m², such as up to about 4 mg/m², such as up to about 3 mg/m². In a further embodiment, vinblastine is administered once weekly, such as once per week in a treatment cycle lasting 5 weeks.

In one embodiment, the total daily dose of pemetrexed is up to about 500 mg/m², such as about 500 mg/m². In a further embodiment, pemetrexed is administered on days 1 of a treatment cycle lasting 21 days.

In one embodiment, the total daily dose of paclitaxel is from about 45 mg/m² to about 200 mg/m², such as up to about 200 mg/m², such as up to about 150 mg/m², such as up to about 100 mg/m², such as up to about 50 mg/m², such as up to about 45 mg/m². In a further embodiment, paclitaxel is administered once weekly, such as once per week in a treatment cycle lasting 21 days. In a further embodiment, paclitaxel is administered on day 1 only of a treatment cycle lasting 21 days.

In one embodiment, the total daily dose of vinorelbine is from about 25 mg/m² to about 30 mg/m², such as up to about 30 mg/m², such as up to about 25 mg/m². In a further embodiment, vinorelbine is administered on day 1 of each treatment cycle. In a further embodiment, vinorelbine is administered on days 1 and 8 of a treatment cycle lasting 21 days. In a further embodiment, vinorelbine is administered on days 1, 8, 15 and 22 of a treatment cycle lasting 28 days.

In one embodiment, the total daily dose of gemcitabine is from about 1000 mg/m² to about 1500 mg/m², such as up to about 1500 mg/m², such as up to about 1250 mg/m², such as up to about 1000 mg/m². In a further embodiment, gemcitabine is administered on day 1 of each treatment cycle. In a further embodiment, gemcitabine is administered on days 1 and 8 of a treatment cycle lasting 21 days.

In one embodiment, the total daily dose of docetaxel is up to about 75 mg/m², such as about 75 mg/m². In a further embodiment, docetaxel is administered on day 1 of each treatment cycle. In a further embodiment, docetaxel is administered on day 1 of a treatment cycle lasting 21 days.

Patients subject to CRT may undergo one or more treatment cycles. In one embodiment, therefore, the platinum-based chemotherapy may be administered on up to 6 treatment cycles, or up to 5 treatment cycles, or up to 4 treatment cycles, or up to 3 treatment cycles, or up to 2 treatment cycles, or in a single treatment cycle.

In one embodiment, the CCRT regimens may be selected from:
a) a 42 day treatment cycle with a total daily dose of cisplatin of about 50 mg/m² on days 1, 8, 29 and 36; a total daily dose of etoposide of about 50 mg/m² on days 1, 2, 3, 4, 5, 29, 30, 31, 32 and 33; and concurrent radiation therapy;
b) a 35 day treatment cycle with a total daily dose of cisplatin of about 100 mg/m² on days 1 and 29; a total daily dose of vinblastine of about 5 mg/m² weekly; and concurrent radiation therapy;
c) a 21 day treatment cycle with a total daily dose of carboplatin of AUC 5 on day 1; a total daily dose of pemetrexed of about 500 mg/m² on day 1; and concurrent radiation therapy; and the total number of treatment cycles may be up to 4 cycles;
d) a 21 day treatment cycle with a total daily dose of cisplatin of about 75 mg/m² on day 1; a total daily dose of pemetrexed of about 500 mg/m² on day 1; and concurrent radiation therapy; the total number of treatment cycles may be up to 3 cycles; and pemetrexed alone may optionally be administered at about 500 mg/m² for a further 4 cycles;
e) a 7 day treatment cycle with a total weekly dose of carboplatin of AUC 2; a total weekly of paclitaxel of about 45-50 mg/m²; and concurrent radiation therapy; and optionally a further 2 cycles with a total weekly dose of carboplatin of AUC 6 and a total weekly of paclitaxel of about 200 mg/m².

In one embodiment, the SCRT regimens may be selected from:
a) a 28 day treatment cycle with a total daily dose of cisplatin of about 50 mg/m² on days 1 and 8; a total daily dose of vinorelbine of about 25 mg/m² on days 1, 8, 15 and 22; the total number of treatment cycles may be up to 4 cycles; and followed by radiation therapy;
b) a 28 day treatment cycle with a total daily dose of cisplatin of about 100 mg/m² on day 1; a total daily dose of vinorelbine of about 30 mg/m² on days 1, 8, 15 and 22; the total number of treatment cycles may be up to 4 cycles; and followed by radiation therapy;
c) a 21 day treatment cycle with a total daily dose of cisplatin of about 75-80 mg/m² on day 1; a total daily dose of vinorelbine of about 25-30 mg/m² on days 1 and 8; the total number of treatment cycles may be up to 4 cycles; and followed by radiation therapy;
d) a 28 day treatment cycle with a total daily dose of cisplatin of about 100 mg/m² on day 1; a total daily dose of etoposide of about 100 mg/m² on days 1, 2 and 3; the total number of treatment cycles may be up to 4 cycles; and followed by radiation therapy;
e) a 21 day treatment cycle with a total daily dose of cisplatin of about 75 mg/m² on day 1; a total daily dose of gemcitabine of about 1250 mg/m² on days 1 and 8; the total number of treatment cycles may be up to 4 cycles; and followed by radiation therapy;
f) a 21 day treatment cycle with a total daily dose of cisplatin of about 75 mg/m² on day 1; a total daily dose of docetaxel of about 75 mg/m² on day 1; the total number of treatment cycles may be up to 4 cycles; and followed by radiation therapy;
g) a 21 day treatment cycle with a total daily dose of cisplatin of about 75 mg/m² on day 1; a total daily dose of pemetrexed of about 500 mg/m² on day 1; the total number of treatment cycles may be up to 4 cycles; and followed by radiation therapy;
h) a 21 day treatment cycle with a total daily dose of carboplatin of AUC 6 on day 1; a total daily dose of paclitaxel of about 200 mg/m² on day 1; the total number of treatment cycles may be up to 4 cycles; and followed by radiation therapy;
i) a 21 day treatment cycle with a total daily dose of carboplatin of AUC 5 on day 1; a total daily dose of gemcitabine of about 1000 mg/m² on days 1 and 8; the total number of treatment cycles may be up to 4 cycles; and followed by radiation therapy; and
j) a 21 day treatment cycle with a total daily dose of carboplatin of AUC 5 on day 1; a total daily dose of pemetrexed of about 500 mg/m² on day 1; the total number of treatment cycles may be up to 4 cycles; and followed by radiation therapy.

### Osimertinib and pharmaceutical compositions thereof

Osimertinib has the following chemical structure:

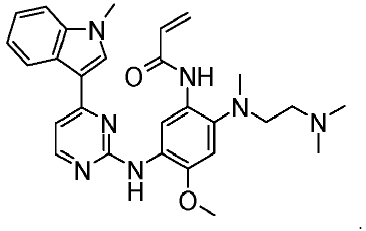

The free base of osimertinib is known by the chemical name: *N*-(2-{2-dimethylamino ethyl-methylamino}-4-methoxy-5-{[4-(1-methylindol-3-yl)pyrimidin-2-yl]amino}phenyl) prop-2-enamide. Osimertinib is described in WO 2013/014448. Osimertinib is also known as AZD9291.

Osimertinib may be found in the form of the mesylate salt: *N*-(2-{2-dimethylamino ethyl-methylamino}-4-methoxy-5-{[4-(1-methylindol-3-yl)pyrimidin-2-yl]amino}phenyl) prop-2-enamide mesylate salt. Osimertinib mesylate is also known as TAGRISSO^{™}.

Osimertinib mesylate is currently approved as an oral once daily tablet formulation, at a dose of 80 mg (expressed as free base, equivalent to 95.4 mg osimertinib mesylate), for the treatment of metastatic EGFR T790M mutation positive NSCLC patients. A 40 mg oral once daily tablet formulation (expressed as free base, equivalent to 47.7 mg osimertinib mesylate) is available should dose modification be required. The tablet core comprises pharmaceutical diluents (such as mannitol and microcrystalline cellulose), disintegrants (such as low-substituted hydroxypropyl cellulose) and lubricants (such as sodium stearyl fumarate). The tablet formulation is described in WO 2015/101791.

In one embodiment, therefore, osimertinib is in the form of the mesylate salt, N-(2-{2-dimethylamino ethyl-methylamino}-4-methoxy-5- f [4-(1-methylindol-3-yl)pyrimidin-2-yl]amino}phenyl) prop-2-enamide mesylate salt.

In a further embodiment, osimertinib or a pharmaceutically acceptable salt thereof is administered once-daily. In a further embodiment, osimertinib mesylate is administered once-daily.

In a further embodiment, the total daily dose of osimertinib is about 80 mg. In a further embodiment, the total daily dose of osimertinib mesylate is about 95.4 mg

In another embodiment, the total daily dose of osimertinib is about 40 mg. In a further embodiment, the total daily dose of osimertinib mesylate is about 47.7 mg.

In a further embodiment, osimertinib is administered in the form of a pharmaceutical composition comprising one or more pharmaceutically acceptable excipients. In a further embodiment, the composition comprises one or more pharmaceutical diluents (such as mannitol and microcrystalline cellulose), one or more pharmaceutical disintegrants (such as low-substituted hydroxypropyl cellulose) or one or more pharmaceutical lubricants (such as sodium stearyl fumarate).

In a further embodiment, the composition is in the form of a tablet, wherein the tablet core comprises: (a) from 2 to 70 parts of osimertinib or a pharmaceutically acceptable salt thereof; (b) from 5 to 96 parts of two or more pharmaceutical diluents; (c) from 2 to 15 parts of one or more pharmaceutical disintegrants; and (d) from 0.5 to 3 parts of one or more pharmaceutical lubricants; and wherein all parts are by weight and the sum of the parts (a)+(b)+(c)+(d)=100.

In a further embodiment, the composition is in the form of a tablet, wherein the tablet core comprises: (a) from 7 to 25 parts of osimertinib or a pharmaceutically acceptable salt thereof; (b) from 55 to 85 parts of two or more pharmaceutical diluents, wherein the pharmaceutical diluents comprise microcrystalline cellulose and mannitol; (c) from 2 to 8 parts of pharmaceutical disintegrant, wherein the pharmaceutical disintegrant comprises low-substituted hydroxypropyl cellulose; (d) from 1.5 to 2.5 parts of pharmaceutical lubricant, wherein the pharmaceutical lubricant comprises sodium stearyl fumarate; and wherein all parts are by weight and the sum of the parts (a)+(b)+(c)+(d)=100.

In a further embodiment, the composition is in the form of a tablet, wherein the tablet core comprises: (a) about 19 parts of osimertinib mesylate; (b) about 59 parts of mannitol; (c) about 15 parts of microcrystalline cellulose; (d) about 5 parts of low-substituted hydroxypropyl cellulose; and (e) about 2 parts of sodium stearyl fumarate; and wherein all parts are by weight and the sum of the parts (a)+(b)+(c)+(d)+(e)=100.

### Dosing Regimens

Osimertinib or a pharmaceutically acceptable salt thereof will be administered to patients with locally advanced unresectable EGFR mutation-positive NSCLC (Stage III), whose disease has not progressed following definitive platinum-based chemoradiation therapy. Progression may be assessed in accordance with the RECIST criteria. The skilled person will be aware of the RECIST criteria and of suitable methods of assessing evidence of disease progression following chemoradiation therapy (Eur. J. Cancer [2009], vol. 45(2), 228-247).

The order of sequencing of the chemoradiation therapy and the treatment with osimertinib may be an important factor in how a given patient responds. In particular, it may be important to administer osimertinib to a patient after chemoradiation therapy is completed, and to avoid the use of osimertinib either prior to, or concurrently with, such chemoradiation therapy.

In one embodiment, therefore, osimertinib is administered to the patient after the completion of definitive platinum-based chemoradiation therapy. In another embodiment, osimertinib is not administered to the patient prior to the completion of definitive platinum-based chemoradiation therapy. In another embodiment, osimertinib is not administered to the patient concurrently with definitive platinum-based chemoradiation therapy. In another embodiment, osimertinib is not administered to the patient either prior to or concurrently with definitive platinum-based chemoradiation therapy.

In a further embodiment, the patient will be in complete response following definitive platinum-based chemoradiation therapy. In a further embodiment, the patient will be in partial response following definitive platinum-based chemoradiation therapy. In a further embodiment, the patient will have stable disease following definitive platinum-based chemoradiation therapy.

It may be preferable not to have a significant time period between the completion of chemoradiation therapy and the first administration of osimertinib.

In one embodiment, therefore, the first dose of osimertinib or a pharmaceutically acceptable salt thereof is administered not later than 12 weeks after the completion of chemoradiation therapy, such as not later than 10 weeks, such as not later than 8 weeks, such as not later than 6 weeks, such as not later than 4 weeks, such as not later than 2 weeks, such as not later than 1 week.

In a further embodiment, osimertinib or a pharmaceutically acceptable salt thereof is administered to the patient until the time of disease progression. In another embodiment, osimertinib or a pharmaceutically acceptable salt thereof is administered to the patient up to a period of five years after the completion of chemoradiation therapy, such as up to 4 years, such as up to 3 years, such as up to 30 months, such as up to 24 months, such as up to 18 months, such as up to 12 months.

Patients with locally advanced unresectable NSCLC (Stage III) who receive osimertinib or a pharmaceutically acceptable salt thereof according to this specification may benefit from an improved prognosis compared to existing standard of care. In particular, such patients may benefit one or more of improved progression free survival (PFS); increased objective response rate; improved duration of response (DoR); or improved overall survival (OS).

In one embodiment, therefore, the patient benefits from progression free survival of at least 12 months, such as at least 14 months, such as at least 16 months, such as at least 18 months, such as at least 20 months, such as at least 22 months, such as at least 24 months, such as at least 26 months, such as at least 28 months, such as at least 30 months, such as at least 32 months, such as at least 34 months, such as at least 36 months. In a further embodiment, the patient benefits from a duration of response of at least 15 months, such as at least 20 months, such as at least 25 months, such as at least 30 months, such as at least 35 months. In a further embodiment, the patient benefits from an overall survival of at least 30 months, such as at least 35 months, such as at least 40 months, such as at least 45 months, such as at least 50 months, such as at least 55 months, such as at least 60 months.

Patients with locally advanced unresectable NSCLC (Stage III) who receive osimertinib or a pharmaceutically acceptable salt thereof according to this specification may particularly benefit from an improved prognosis compared to existing standard of care in the incidence and/or progression of central nervous system metastases, and in particular brain metastases.

In one embodiment, therefore, the patient selected for treatment with osimertinib or a pharmaceutically acceptable salt thereof according to this specification is at risk of developing central nervous system metastases, such as brain metastases.

In a further embodiment, the osimertinib or a pharmaceutically acceptable salt thereof is provided for use in reducing the incidence of central nervous system metastases, such as brain metastases, in the patient.

In a further embodiment, the osimertinib or a pharmaceutically acceptable salt thereof is provided for use in improving one or more of progression free survival (PFS); duration of response (DoR); or overall survival (OS) in a patient at risk of developing central nervous system metastases, such as brain metastases.

In a further embodiment, the osimertinib or a pharmaceutically acceptable salt thereof is provided for use in improving one or more of progression free survival (PFS); duration of response (DoR); or overall survival (OS) in a patient who develops central nervous system metastases, such as brain metastases.

In a further embodiment, the patient benefits from central nervous system progression free survival of at least 12 months, such as at least 14 months, such as at least 16 months, such as at least 18 months, such as at least 20 months, such as at least 22 months, such as at least 24 months, such as at least 26 months, such as at least 28 months, such as at least 30 months, such as at least 32 months, such as at least 34 months, such as at least 36 months.

### Further embodiments

In one embodiment, there is provided osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily.

In a further embodiment, there is provided a method of treating locally advanced unresectable EGFR mutation-positive NSCLC (Stage III) in a human patient comprising administering to the patient osimertinib or a pharmaceutically acceptable salt thereof once-daily wherein the disease has not progressed following definitive platinum-based chemoradiation therapy.

In a further embodiment, there is provided the use of osimertinib or a pharmaceutically acceptable salt thereof in the manufacture of a medicament for the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily.

In one embodiment, there is provided osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the platinum-based chemoradiation therapy comprises platinum-based doublet chemotherapy.

In a further embodiment, there is provided a method of treating locally advanced unresectable EGFR mutation-positive NSCLC (Stage III) in a human patient comprising administering to the patient osimertinib or a pharmaceutically acceptable salt thereof once-daily wherein the disease has not progressed following definitive platinum-based chemoradiation therapy, and wherein the platinum-based chemoradiation therapy comprises platinum-based doublet chemotherapy.

In a further embodiment, there is provided the use of osimertinib or a pharmaceutically acceptable salt thereof in the manufacture of a medicament for the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the platinum-based chemoradiation therapy comprises platinum-based doublet chemotherapy.

In one embodiment, there is provided osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the total dose of radiation administered to the patient as part of the chemoradiation therapy is up to about 100 Gy.

In a further embodiment, there is provided a method of treating locally advanced unresectable EGFR mutation-positive NSCLC (Stage III) in a human patient comprising administering to the patient osimertinib or a pharmaceutically acceptable salt thereof once-daily wherein the disease has not progressed following definitive platinum-based chemoradiation therapy, and wherein the total dose of radiation administered to the patient as part of the chemoradiation therapy is up to about 100 Gy.

In a further embodiment, there is provided the use of osimertinib or a pharmaceutically acceptable salt thereof in the manufacture of a medicament for the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the total dose of radiation administered to the patient as part of the chemoradiation therapy is up to about 100 Gy.

In one embodiment, there is provided osimertinib or a pharmaceutically acceptable salt thereof for use in the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the EGFR mutation-positive NSCLC comprises activating mutations in EGFR selected from exon 19 deletions or L858R substitution mutations.

In a further embodiment, there is provided a method of treating locally advanced unresectable EGFR mutation-positive NSCLC (Stage III) in a human patient comprising administering to the patient osimertinib or a pharmaceutically acceptable salt thereof once-daily wherein the disease has not progressed following definitive platinum-based chemoradiation therapy, and wherein the EGFR mutation-positive NSCLC comprises activating mutations in EGFR selected from exon 19 deletions or L858R substitution mutations.

In a further embodiment, there is provided the use of osimertinib or a pharmaceutically acceptable salt thereof in the manufacture of a medicament for the treatment of patients with locally advanced unresectable EGFR mutation-positive NSCLC (Stage III), whose disease has not progressed following definitive platinum-based chemoradiation therapy, wherein the osimertinib or a pharmaceutically acceptable salt thereof is administered once-daily, and wherein the EGFR mutation-positive NSCLC comprises activating mutations in EGFR selected from exon 19 deletions or L858R substitution mutations.

### EXAMPLES

### A Phase III, randomized, double-blind, placebo-controlled, multicenter, international study of osimertinib as maintenance therapy in patients with locally advanced, unresectable EGFR mutation-positive NSCLC (Stage III) whose disease has not progressed following definitive platinum based chemoradiation therapy.

The following study is carried out to confirm the benefit of osimertinib in patients with locally advanced, unresectable EGFR mutation-positive NSCLC (Stage III) whose disease has not progressed following definitive platinum based chemoradiation therapy.

### Study site(s) and number of patients planned

Study sites ~70. Approximately 200 patients will be randomised (patients will be in complete response [CR], partial response [PR] or have stable disease [SD] following definitive platinum-based, chemoradiation therapy).

### Study design

A Phase III, randomized, double-blind, placebo-controlled, multicenter international study assessing the efficacy and safety of osimertinib compared with placebo, as maintenance therapy in patients with locally advanced, unresectable epidermal growth factor receptor mutation positive non-small cell lung cancer (Stage III), whose disease has not progressed following definitive platinum based chemoradiation therapy.

### Investigational product, dosage and mode of administration

Osimertinib 80 mg or matching placebo will be administered orally, once daily.

### Study objectives

| **Primary objective** | **Outcome measure** |
|---|---|
| To assess the efficacy of osimertinib treatment compared with placebo in terms of PFS. | PFS using BICR assessment according to RECIST v 1.1. |
| | Sensitivity analysis of PFS using Investigator assessment according to RECIST v1.1 |

| **Secondary objective** | **Outcome measure** |
|---|---|
| To further assess the efficacy of | OS |
| osimertinib compared with placebo in terms of: | ORR, DoR, DCR and tumor shrinkage, TTDM using BICR assessments according to RECIST v1.1 |
| • OS; | |
| • ORR; | |
| • DoR; | |
| • DCR; | |
| • Tumor shrinkage; | |
| • Time to death or distant metastases (TTDM) | |
| To assess the efficacy of osimertinib compared with placebo by assessment of PFS in patients with: | PFS using BICR assessment according to RECIST v 1. 1. |
| | Sensitivity analysis of PFS using Investigator assessment according to RECIST v1.1. |
| - Positive (or negative) pretreatment T790M (amino acid substitution at position 790 in EGFR, from a threonine to a methionine) mutation. | |
| - EGFR Ex19del or L858R mutation. | |
| - EGFRm+ Ex19del or L858R detectable in plasma-derived circulating tumour deoxyribonucleic acid (ctDNA) | |
| To assess the efficacy of osimertinib versus placebo on PFS CNS | Time to PFS CNS (time to the earliest of CNS progression or death) using BICR assessments according to RECIST v1.1. |
| | Cumulative incidence rate of PFS CNS by BICR at 12 and 24 months . |
| To further assess the efficacy of osimertinib compared to Placebo post progression. | Second progression free survival (PFS2). |
| | Time to first subsequent therapy (TFST) |
| | Time to second subsequent therapy (TSST) |
| To assess disease-related symptoms and health-related QoL in patients treated with osimertinib compared with placebo using EORTC QLQC30 and EORTC QLQ-LC13. | Change in patient-reported symptoms, functioning, and global health status/QoL |
| To assess the safety and tolerability profile of osimertinib compared with placebo. | • AEs (graded by CTCAE v4); |
| | • Clinical chemistry, hematology and urinalysis; |
| | • Vital signs (pulse and blood pressure), physical examination, weight; |
| | • Centrally reviewed digital ECG; |
| | • Echocardiogram/MUGA scan (for left ventricular ejection fraction); |
| | • WHO Performance Status. |
| To assess the PK of osimertinib. | Trough plasma concentrations of osimertinib, and its metabolite AZ5104. |
| | If conducted, PK Parameters (CL_{ss/F}, Cₛₛ, ₘᵢₙ and C_{ss, max}, AUCₛₛ) may be derived using population PK analysis and reported separately to the CSR. Data from this study may form part of a pooled analysis with data from other studies. Trough plasma concentrations of osimertinib, and its metabolite AZ5104. |

### Screening

The study will include a 2-part screening process. In Part I, following signing of the Part I screening consent form, the patient's archival tumor sample will be tested in a central laboratory for EGFR mutation status to determine patient eligibility. In addition a plasma sample for assessment of EGFR mutations for retrospective testing will be collected. No other screening assessments will be performed during Part I. Part 1 screening can occur before, during or after chemoradiation. If the tissue EGFR mutation test is positive for Ex19Del or L858R mutations patients will enter Part II of screening, after completion of chemoradiation and following signing of the main consent form, and will begin to complete Visit 1 assessments when they are confirmed eligible for consideration.

Patients that have a pre-existing EGFR mutation positive test result using the cobas^{®} EGFR Mutation Test v2 performed in Clinical Laboratory Improvement Amendments (CLIA)-certified (for US sites) or accredited laboratories (outside of the US) can enter Part II screening directly.

### Target patient population

### KEY INCLUSION CRITERIA

Male and Female patients must be aged at least 18 years. Patients from Japan aged at least 20 years.

Patients with histologically documented NSCLC of predominantly non-squamous pathology who present with locally advanced, unresectable NSCLC (Stage III) (according to Version 8 of the International Association for the Study of Lung Cancer [IASLC] Staging Manual in Thoracic Oncology 2016) whose tumors have EGFR exon 19 deletions or exon 21 (L858R) substitution mutations, either alone or in combination with other EGFR mutations, as detected by a cobas^{®} EGFR Mutation Test v2 (Roche Diagnostics) of tissue.

Except for overt cT4 disease, nodal status N2 or N3 should have been proven by biopsy, via endobronchial ultrasound, mediastinoscopy, or thoracoscopy. In absence of biopsy, nodal status should have been confirmed with whole body ¹⁸F-fluoro-deoxyglucose positron emission tomography (PET) plus contrast-enhanced computed tomography (CT) in addition to or in combination with PET.

Patients must not have had disease progression during or following definitive platinum-based, chemoradiation therapy. Both concurrent chemoradiation (CCRT) regimens and sequential chemoradiation (SCRT) are permitted as defined below:
CCRT- Patients must have received at least 2 cycles of platinum-based chemotherapy (or 5 doses of weekly platinum-based doublet regimen) concurrent with radiation therapy, which must be completed within 6 weeks prior to the first dose of IP in the study.

Administration of chemotherapy prior to CCRT is permitted. The final chemotherapy cycle must end prior to, or concurrently with, the final dose of radiation. Consolidation chemotherapy after radiation is not permitted but administration of chemotherapy prior to CCRT is permitted.

SCRT- SCRT is defined as chemotherapy followed by radiation therapy and not radiation therapy followed by chemotherapy. Patients must have received at least 2 cycles of platinum-based chemotherapy (or 5 doses of weekly platinum-based doublet regimen) prior to radiation treatment, which must be completed within 6 weeks prior to the first dose of IP in the study. Consolidation chemotherapy after radiation is not permitted.

If a patient has received at least 2 cycles of platinum-based chemotherapy (or 5 doses of weekly platinum-based chemotherapy) and subsequently receives one cycle of platinum-based chemotherapy or < 5 doses of weekly platinum-based chemotherapy concurrent with radiation therapy, this will be considered SCRT.

If a patient has received 1 cycle of platinum-based chemotherapy (or <5 doses of weekly platinum-based chemotherapy) and subsequently receives one cycle of platinum-based chemotherapy or < 5 doses of weekly platinum-based doublet regimen concurrent with radiation therapy, this will not be considered CCRT or SCRT and the patient will not be eligible.

The platinum-based doublet chemotherapy regimen must contain one of the following agents: etoposide, vinblastine, vinorelbine, a taxane (paclitaxel or docetaxel), or pemetrexed, according to the local standard of care regimens. Gemcitabine is permitted if used prior to radiation but not with radiation.

Patients final chemotherapy cycle must end prior to, or concurrently with, the final dose of radiation.

Patients must have received a total dose of radiation of 60 Gy ± 10% (54 to 66 Gy) as part of the chemoradiation therapy. It is recommended but not required that patients eligible for randomization have a
- Mean lung dose <20 Gy and/or V20 <35%
- Mean esophagus dose <34 Gy
- Heart V50 ≤25%, V30 ≤50%, and V45 ≤35

Patients must have World Health Organization (WHO) PS of 0 or 1 at enrollment and randomization.

### Adequate Organ Function

### KEY EXCLUSION CRITERIA

Patient has history of symptomatic ILD prior to chemoradiation.

Patient has symptomatic pneumonitis following chemoradiation.

Patient with any unresolved toxicity Common Terminology Criteria for Adverse Events (CTCAE) > Grade 2 from the prior chemoradiation therapy. Patient with irreversible toxicity that is not reasonably expected to be exacerbated by study drug may be included (e.g. hearing loss) after consultation with the AstraZeneca medical monitor.

### Study assessments and patient follow-up

Randomization must occur within 6 weeks of completion of radiation and the screening period will be performed within 28 days of the start of study treatment. Following randomization patients will have visits at week 2, week 4 and every 4 weeks until week 24, every 8 weeks till week 48 and then every 12 weeks until discontinuation from study drug. Osimertinib (80 mg orally, once daily) or matching placebo, in accordance with the randomization schedule, will be administered orally once daily.

All subjects should continue to receive randomized study treatment until objective radiographic disease progression as assessed by BICR, or until a treatment discontinuation criterion is met. Tumor assessments will be performed using (i) contrast enhanced computed tomography (CT) of the chest and abdomen (including liver and adrenal glands). However, if subjects are contraindicated to CT contrast agents a non-contrast CT or MRI will be acceptable and (ii) contrast enhanced T1w magnetic resonance imaging (MRI) of the brain. However in those subjects who are contraindicated to gadolinium-diethylenetriamine penta-acetic acid (Gd-DTPA) based contrast agents a non-contrast MRI would be sufficient. CT of chest/abdomen and MRI of the brain will be performed at all tumor imaging visits. The baseline assessment is part of the screening procedures. Objective tumor assessments will be made every 8 weeks (relative to the date of randomization) till 48 weeks, then every 12 weeks thereafter, until objective radiological disease progression occurs as defined by Response Evaluation Criteria in Solid Tumors (RECIST) v1.1 and as confirmed by BICR (irrespective of the reason for stopping study drug and/or subsequent therapy). Additional scans should be performed if clinically indicated, ie, if disease progression is suspected. If an unscheduled assessment is performed, and the patient's disease has not progressed, every attempt should be made to perform the subsequent assessments at their scheduled visits.

Safety monitoring includes collection of adverse events, assessment of physical examination, vital signs, clinical chemistry, haematology, urinalysis and ECG at all visits. Left ventricular ejection fraction (via ECHO or MUGA) will be conducted periodically.

### Patient -Reported Outcomes

Patient-reported symptoms, functioning, and global health status/QoL will be assessed periodically during administration of study drug and following disease progression.

### Pharmacokinetic Assessments

### Trough PK samples will be taken at weeks 4, 12 and 24

The treatment code should not be broken except in medical emergencies when the appropriate management of the patient requires knowledge of the treatment randomization. Additionally, at the request of the Investigator, at BICR confirmed progression of disease, the patient can be unblinded if considered essential for the future management of the patient. For those patients randomized to the placebo arm, no formal cross-over to the osimertinib arm is permitted. Treatments received by the patient after relapse will be determined by the physician. Post recurrence cancer treatments and procedures will be recorded.

Following discontinuation of trial therapy patients will be followed up until death or withdrawal of consent for assessments of post-progression outcomes (PFS2, TFST, TSST) and for assessment of overall survival.

### Clauses

The specification may be further defined by the following clauses:
Clause 1. Osimertinib or a pharmaceutically acceptable salt thereof for use in the treatment of patients with locally advanced unresectable EGFR mutation-positive NSCLC, whose disease has not progressed following definitive platinum-based chemoradiation therapy.
Clause 2. The compound for use according to clause 1, wherein the unresectable EGFR mutation-positive NSCLC is considered curable by chemoradiation therapy.
Clause 3. The compound for use according to clause 1 or clause 2, wherein the chemoradiation therapy comprises concurrent chemoradiation therapy.
Clause 4. The compound for use according to any preceding clause, wherein the chemoradiation therapy comprises sequential chemoradiation therapy.
Clause 5. The compound for use according to any preceding clause, wherein the chemoradiation therapy comprises a platinum-based doublet chemotherapy.
Clause 6. The compound for use according to any preceding clause, wherein the chemoradiation therapy comprises a platinum-based agent selected from cisplatin or carboplatin.
Clause 7. The compound for use according to any preceding clause, wherein the chemoradiation therapy comprises a non-platinum based agent selected from etoposide, vinorelbine, vinblastine, pemetrexed, paclitaxel, docetaxel, or gemcitabine.
Clause 8. The compound for use according to any preceding clause, wherein the EGFR mutation-positive NSCLC comprises activating mutations in EGFR selected from exon 19 deletions or L858R substitution mutations.
Clause 9. The compound for use according to any preceding clause, wherein the osimertinib or a pharmaceutically acceptable salt thereof is administered once-daily.
Clause 10. The compound for use according to any preceding clause, wherein the osimertinib or a pharmaceutically acceptable salt thereof is not administered to the patient prior to the completion of the chemoradiation therapy.
Clause 11. The compound for use according to any preceding clause, wherein the osimertinib or a pharmaceutically acceptable salt thereof is in tablet form.
Clause 12. The compound for use according to any preceding clause, wherein the osimertinib or a pharmaceutically acceptable salt thereof is in the form of the mesylate salt.
Clause 13. A method of treating locally advanced Stage III unresectable EGFR mutation-positive NSCLC in a human patient comprising administering to the patient osimertinib or a pharmaceutically acceptable salt thereof, wherein the disease has not progressed following definitive platinum-based chemoradiation therapy.
Clause 14. The use of osimertinib or a pharmaceutically acceptable salt thereof in the manufacture of a medicament for the treatment of patients with locally advanced unresectable EGFR mutation-positive NSCLC, whose disease has not progressed following definitive platinum-based chemoradiation therapy.

## Claims

1. Osimertinib or a pharmaceutically acceptable salt thereof for use in the treatment of patients with locally advanced unresectable EGFR mutation-positive NSCLC, whose disease has not progressed following definitive platinum-based chemoradiation therapy.

2. The compound for use according to claim 1, wherein the unresectable EGFR mutation-positive NSCLC is considered curable by chemoradiation therapy.

3. The compound for use according to claim 1 or claim 2, wherein the chemoradiation therapy comprises concurrent chemoradiation therapy.

4. The compound for use according to any preceding claim, wherein the chemoradiation therapy comprises sequential chemoradiation therapy.

5. The compound for use according to any preceding claim, wherein the chemoradiation therapy comprises a platinum-based doublet chemotherapy.

6. The compound for use according to any preceding claim, wherein the chemoradiation therapy comprises a platinum-based agent selected from cisplatin or carboplatin.

7. The compound for use according to any preceding claim, wherein the chemoradiation therapy comprises a non-platinum based agent selected from etoposide, vinorelbine, vinblastine, pemetrexed, paclitaxel, docetaxel, or gemcitabine.

8. The compound for use according to any preceding claim, wherein the EGFR mutation-positive NSCLC comprises activating mutations in EGFR selected from exon 19 deletions or L858R substitution mutations.

9. The compound for use according to any preceding claim, wherein the osimertinib or a pharmaceutically acceptable salt thereof is administered once-daily.

10. The compound for use according to any preceding claim, wherein the osimertinib or a pharmaceutically acceptable salt thereof is not administered to the patient prior to the completion of the chemoradiation therapy.

11. The compound for use according to any preceding claim, wherein the osimertinib or a pharmaceutically acceptable salt thereof is in tablet form.

12. The compound for use according to any preceding claim, wherein the osimertinib or a pharmaceutically acceptable salt thereof is in the form of the mesylate salt.

13. A method of treating locally advanced Stage III unresectable EGFR mutation-positive NSCLC in a human patient comprising administering to the patient osimertinib or a pharmaceutically acceptable salt thereof, wherein the disease has not progressed following definitive platinum-based chemoradiation therapy.

14. The use of osimertinib or a pharmaceutically acceptable salt thereof in the manufacture of a medicament for the treatment of patients with locally advanced unresectable EGFR mutation-positive NSCLC, whose disease has not progressed following definitive platinum-based chemoradiation therapy.
